(19) Europäisches Patentamt
European Patent Office
Office européen des brevets

(11) Publication number: **0 302 379 B1**

# (12) EUROPEAN PATENT SPECIFICATION

(45) Date of publication of patent specification: **29.09.93**

(51) Int. Cl.[5]: **C07D 281/08**, A61K 31/55

(21) Application number: **88112134.7**

(22) Date of filing: **27.07.88**

The file contains technical information submitted after the application was filed and not included in this specification

## (54) Naphthothiazepine derivatives and a process for preparing the same.

(30) Priority: **31.07.87 JP 193131/87**

(43) Date of publication of application:
**08.02.89 Bulletin 89/06**

(45) Publication of the grant of the patent:
**29.09.93 Bulletin 93/39**

(84) Designated Contracting States:
**AT BE CH DE ES FR GB GR IT LI LU NL SE**

(56) References cited:
**EP-A- 0 154 838**
**EP-A- 0 182 273**
**EP-A- 0 320 817**
**US-A- 4 652 561**

(73) Proprietor: **TANABE SEIYAKU CO., LTD.**
**2-10, Dosho-machi 3-chome**
**Chuo-ku Osaka(JP)**

(72) Inventor: **Inoue, Hirozumi**
**No. 8-16, Oizumi-gakuencho 6-chome**
**Nerima-ku Tokyo-to(JP)**
Inventor: **Gaino, Mitsunori RC-5-306 Sunadan-chi**
**No. 4-1, Higashi-Omiya 3-chome**
**Omiya-shi Saitama-ken(JP)**
Inventor: **Nagao, Taku**
**No. 36-21, Kamimeguro 4-chome**
**meguro-ku Tokyo-to(JP)**
Inventor: **Murata, Sakae**
**No. 5-15, Kasumigaseki-kita 3-chome**
**Kawagoe-shi Saitama-ken(JP)**

(74) Representative: **Hansen, Bernd, Dr.rer.nat. et al**
**Hoffmann, Eitle & Partner, Patentanwälte,**
**Postfach 81 04 20**
**D-81904 München (DE)**

EP 0 302 379 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel naphthothiazepine derivatives, and processes for preparing same. More particularly, it relates to cis naphthothiazepine derivatives of the formula (I):

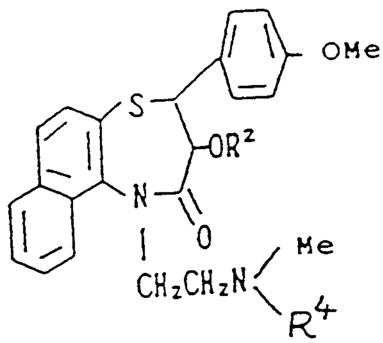

wherein $R^2$ is a hydrogen atom or an acetyl group, and $R^4$ is a methyl group, or a pharmaceutically acceptable acid addition salt thereof.

U.S. Patent 4,652,561 discloses that naphthothiazepine derivatives such as (±)-cis-2-(4-methoxyphenyl)-3-acetoxy-5-[2-(dimethylamino)ethyl]-2,3-dihydro-naphtho[1,2-b][1,5] thiazepin-4(5H)-one show hypotensive effect and are useful as calcium channel blockers.

As compared with said known compounds, the novel naphthothiazepine derivative (I) of the present invention and a salt thereof show stronger hypotensive and vasodilating effects and are more useful for treatment or prophylaxis of various vascular diseases.

According to the present invention, the compound (I) can be prepared by condensing a compound of the formula:

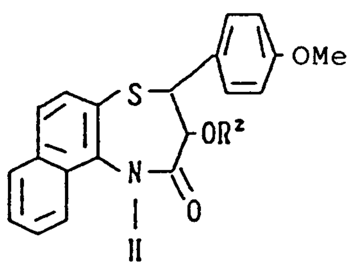

(II)

wherein $R^2$ is the same as defined above, or a salt thereof with a compound of the formula:

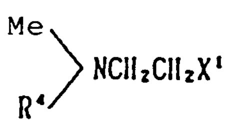

(III)

wherein $R^4$ is the same as defined above, and $X^1$ is a halogen atom, or a salt thereof.

The compound (I) of the present invention in which $R^2$ is an acetyl group can also be prepared by condensing a compound of the formula:

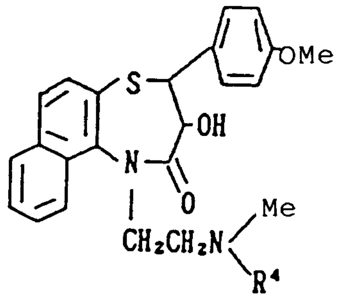

(I-A)

wherein $R^4$ is the same as defined above, or a salt thereof with a compound of the formula:

Me COOH (IV)

or a reactive derivative thereof.

The starting compound (II) may, if required, be used for the above reaction in the form of an alkali metal salt (e.g., sodium salt or potassium salt), and the starting compound (III) and the compound (I-A), may, if required, be used for the above reaction in the form of an acid addition salt such as an inorganic acid addition salt (e.g., hydrochloride or hydrobromide) or an organic acid addition salt (e.g., maleate, fumarate or methanesulfonate).

The condensation of the compound (II) or a salt thereof with the compound (III) or a salt thereof can be carried out in the presence or absence of an alkali reagent in a suitable solvent. When the compound (II) is used in the free form, the said condensation is preferably carried out in the presence of an alkali reagent. Suitable examples of the alkali reagent include an alkali metal hydroxide (e.g., sodium hydroxide, potassium hydroxide), an alkali metal carbonate (e.g., sodium carbonate, potassium carbonate) or an alkali metal hydride (e.g., sodium hydride ). It is preferred to carry out the condensation at 20 to 100 °C, especially 25 to 70 °C.

The condensation of the compound (I-A) or a salt thereof with the compound (IV) can be carried out in the presence of a condensing agent in a suitable solvent. Suitable examples of the condensing agent include dicyclohexylcarbodiimide, N,N'-diisopropylcarbodiimide or N,N'-di-p-tolylcarbodiimide. It is preferred to carry out the reaction at 0 to 30 °C.

On the other hand, the condensation of the compound (I-A) with the reactive derivative of the compound (IV) can be carried out in the presence or absence of an acid acceptor in a suitable solvent. Suitable examples of the reactive derivative include the corresponding acid halides (e.g., chloride, bromide), acid anhydrides, mixed acid anhydrides (e.g., ethoxycarbonyl ester, isopropoxycarbonyl ester, isobutoxycarbonyl ester) or active esters (e.g., p-nitrophenyl ester, 2,4,5-trichlorophenyl ester, 2,4,6-trichlorophenyl ester, N-hydroxysuccinimide ester, 1-benzotriazol ester). Suitable examples of the acid acceptor include pyridine, triethylamine, an alkali metal carbonate, an alkali metal bicarbonate or an alkali metal hydroxide. It is preferred to carry out the reaction at a temperature of 0 to 100 °C.

Suitable examples of the solvent which may preferably used in the above-mentioned reactions are benzene, dioxane, methylene chloride, chloroform, acetone, acetonitrile, ethyl acetate or dimethylformamide.

The compound (I) of the present invention can exist in the form of two optical isomers [i.e., (+)-cis, (-)-cis isomers] because of two asymmetric carbon atoms at the 2- and 3- positions of naphthothiazepine skeleton. However, since all the above-mentioned reactions proceed without racemization, the compound (I) of the present invention in an optically active form can easily be obtained by using an optical isomer of the compound (II) as the starting compound.

The thus-obtained compound (I) of the present invention can be used for pharmaceutical purpose either in the free form or in the form of a pharmaceutically acceptable acid addition salt thereof. Examples of the salt of the compound (I) include inorganic acid addition salts such as hydrochloride, hydrobromide, hydroiodide, perchlorate, sulfate, or phosphate, and organic acid addition salts such as oxalate, maleate, fumarate, tartrate, methanesulfonate, or succinate. These salts can be readily obtained, for example, by treating the compound (I) with an acid in conventional manners.

Because of potent hypotensive, and cerebral and coronary vasodilating effects, the compound (I) of the present invention and a pharmaceutically acceptable acid addition salt are useful for the treatment and prophylaxis of hypotension, cerebral diseases such as cerebrovascular spasm, cerebral ischemia and

cerebral infarction, and heart diseases such as angina pectoris and myocardial infarction.

Moreover, the compound (I) of the present invention and a salt thereof are characteristic in that they have less unfavorable side effects such as less inhibition of atrioventricular conduction or less decrease in heart rate, though known vasodilators such as Verapanil have been known to prolong the atrioventricular conduction time by inhibiting the atrioventricular conduction and sometimes induce arrhythmia due to such conduction disturbance.

Further, the compound (I) of the present invention and a salt thereof have a potent platelet aggregation inhibitory effect and is much more useful for the treatment of cerebral diseases such as cerebrovascular spasm, cerebral ischemia and cerebral infarction.

The compound (I) of the present invention or a pharmaceutically acceptable acid addition salt can be used in the form of a pharmaceutical preparation which may contain pharmaceutical excipients suitable for oral or parenteral administration. Suitable examples of the excipient include starch, lactose, glucose, potassium phosphate, corn starch, acacia, stearic acid, and other conventional pharmaceutical excipients. The pharmaceutical preparation may be used in the solid dosage form such as tablets, pills, capsules or suppositories or in the liquid dosage form such as solutions, suspensions or emulsions. Moreover, when administrated parenterally, the pharmaceutical preparation may be used in the form of injections.

The daily dose of the compound (I) of the present invention or a pharmaceutically acceptable salt thereof may vary depending on the administration route, the age, body weight or conditions of patients and the severities of diseases to be treated. In general, however, preferred daily dose of the compound (I) or a salt thereof is usually in the range of 0.05 to 10 mg/kg, especially about 0.5 to 10 mg/kg in case of oral administration, or 0.05 to 2 mg/kg in case of parenteral administration (e.g., intravenous injection).

Concomitantly, the starting compound (II) of the present invention can be prepared, for example, by condensing 1-aminonaphthalene-2-thiol with a glycidic ester of the formula:

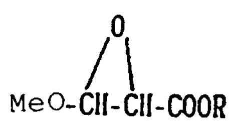

wherein R is an ester residue.

<u>Experiment 1</u>

<u>Hypotensive activity:</u>

(Method)

A test compound dissolved or suspended in water was administered orally at a dose of 30 mg/kg to spontaneously hypotensive rats (SHR) fasted overnight, and the systolic blood pressure of the rats was measured at intervals by the tail plethysmographic technique (The Journal of Laboratory and Clinical Medicine, Vol. 78(1971), page 957). The hypotensive activity of the test compound was estimated in terms of "Decrease in Blood pressure (◁mm Hg)" which was calculated by the following formula:

**Decrease in Blood pressure (Δmm Hg) =**

**[Blood pressure measured immediately before administration of a test compound] - [Blood pressure measured after administration of a test compound]**

(Results)

The results are shown in the following Table 1.

**Table 1**

| | Decrease in Blood pressure (Δmm Hg) | | |
|---|---|---|---|
| | Time after dosing 1 hr | 2 hrs | 5 hrs |
| 1. Test compound*) | 58.3±8.8 | 51.3±7.7 | 34.0±6.2 |

*): Chemical name of the test compound: (±)-cis-2-(4-methoxyphenyl)-3-acetoxy-5-(2-dimethylamino)ethyl)-2,3-dihydro-naphtho(2,1-b)(1,5)thiazepin-4(5H)-one fumarate

Example 1

(1) A mixture of 7.86 g of 1-aminonaphthalene-2-thiol, 12.16 g of methyl 3-(4-methoxyphenyl)glycidate and 80 ml of toluene is heated at 100 °C. After the reaction is completed, the reaction mixture is cencentrated. Diisopropyl ether is added to the oily residue. The precipitated crystals are collected by filtration, and recrystallized from methanol to give 6.49 g (38%) of methyl (±)-threo-3-(1-amino-2-naphthyl)thio-3-(4-methoxyphenyl)-2-hydroxypropionate.
M.p. 131.5 - 135 °C
Mass(m/e):383($M^+$)
IR(Nujol,$cm^{-1}$):3500,3420,3120,1730
(2) A solution of 4.0 g of the product obtained above in 2.60 g of 5% an aqueous sodium hydroxide solution, 10 ml of ethanol and 2 ml of tetrahydrofuran is stirred at room temperature for 2 hours, acidified (pH 2-3) with 10 % of hydrochloric acid, and extracted with chloroform. The extracts are combined, washed with water, dried, and concentrated. The residue is recrystallized from ethanol to give 3.08 g of (±)-threo-3-(1-amino-2-naphthyl)thio-3-(4-methoxyphenyl)-2-hydroxypropionic acid (M.p. 164 - 165 °C (dec.)). A suspension of 2.8 g of the product obtained above in 140 ml of xylene is refluxed for 7 hours. After cooling, the precipitated crystals are recrystallized from a mixture of chloroform and ethanol to give 1.0 g of (±)-cis-2-(4-methoxyphenyl)-3-hydroxy-2,3-dihydro-naphtho[2,1-b][1,5]thiazepin-4(5H)-one.
M.p. 263 - 267 °C
Mass(m/e):351($M^+$)
IR(Nujol,$cm^{-1}$):3520,3200,3100,3080,3060,1660
(3) A mixture of 3.52 g of the product obtained above, 1.17 g of 2-(dimethylamino)ethyl chloride hydrochloride and 3.24 g of potassium carbonate is refluxed in 135 ml of acetone. After the reaction is completed, the inorganic materials are filtered off, and the filtrate is concentrated under reduced pressure. The residue is dissolved in chloroform, washed with water, dried, and concentrated to dryness. Purification of the thus-obtained residue by silica gel column chromatography [solvent: a mixture of chloroform and methanol (30:1)] gives 2.01 g of (±)-cis-2-(4-methoxyphenyl)-3-hydroxy-5-[2-(dimethylamino)ethyl]-2,3-dihydro-naphtho[2,1-b][1,5]thiazepin-4(5H)-one.

This product is treated with fumaric acid and recrystallized from ethanol to give the corresponding fumarate.
M.p. 216 - 218 °C (decomposition)
Mass(m/e):422($M^+$)
IR(Nujol,$cm^{-1}$):3460,3050,1730,1660

Example 2

2.32 g of (±)-cis-2-(4-methoxyphenyl)-3-hydroxy-5-[2-(dimethylamino)ethyl]-2,3-dihydro-naphtho[2,1-b]-[1,5]thiazepin-4(5H)-one is added to 70 ml of acetic acid-acetic anhydride (1:1), and the mixture is refluxed. After the reaction is completed, the mixture is concentrated under reduced pressure. The residue is dissolved in a small amount of ethanol, and 1.1 equivalents of fumaric acid is added to the solution. The precipitated crystals are recrystallized from ethanol to give 2.0 g of (±)-cis-2-(4-methoxyphenyl)-3-acetoxy-5-[2-(dimethylamino)ethyl]-2,3-dihydro-naphtho[2,1-b][1,5]thiazepin-4(5H)-one fumarate.
M.p. 216 - 218.5 °C (decomposition)
Mass(m/e):464($M^+$)
IR(Nujol,$cm^{-1}$):3060,3040,2600-2300,1740,1690

**Claims**

**Claims for the following Contracting States : AT, BE, CH, DE, FR, GB, IT, LI, LU, NL, SE**

**1.** A cis naphthothiazepine derivative of the formula:

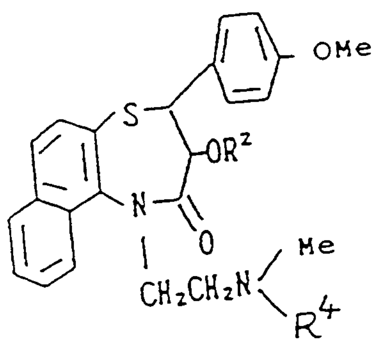

wherein $R^2$ is a hydrogen atom or an acetyl group, and $R^4$ is a methyl group, or a pharmaceutically acceptable acid addition salt thereof.

**2.** A cis naphthothiazepine derivative according to Claim 1 which is (±)-Cis-2-(4-methoxyphenyl)-3-acetoxy 5-[2-(dimethylamino)ethyl]-2,3-dihydro-naphtho[2,1-b][1,5] thiasepin-4(5H)-one, or a pharmaceutically acceptable acid addition salt thereof.

**3.** A cis naphthothiazepine derivative according to Claim 1 which is (±)-Cis-2-(4-methoxyphenyl)-3-hydroxy-5-[2-(dimethylamino)ethyl]-2,3-dihydro-naphtho[2,1-b][1,5]thiazepin-4(5H)-one, or a pharmaceutically acceptable acid addition salt thereof.

**4.** A process for preparing a cis naphthothiazepine derivative according to Claim 1 which is Cis-2-(4-methoxyphenyl)-3-hydroxy-5-[2-(dimethylamino)ethyl]-2,3-dihydro-naphthol[2,1-b][1,5]thiazepin-4(5H)-one, or a pharmaceutically acceptable acid addition salt thereof, which comprises condensing Cis-2-(4-methoxyphenyl)-3-hydroxy-2,3-dihydro-naphtho[2,1-b][1,5]thiazepin-4(5H)-one or a salt thereof with 2-(dimethylamino)ethyl chloride or a salt thereof, and if required, further converting the product into a pharmaceutically acceptable acid addition salt thereof.

**5.** A process for preparing a cis naphthothiazepine derivative according to Claim 1 which is Cis-2-(4-methoxyphenyl)-3-acetoxy-5-[2-(dimethylamino)ethyl]-2,3-dihydro-naphtho[2,1-b][1,5]thiazepin-4(5H)-one, or a pharmaceutically acceptable acid addition salt thereof, which comprises condensing Cis-2-(4-methoxyphenyl)-3-hydroxy-5-[2-(dimethylamino)ethyl]-2,3-dihydro-naphtho-[2,1-b][1,5]thiazepin-4(5H)-

one, or a salt thereof with acetic acid, or a reactive derivative thereof, and if required, further converting the product into a pharmaceutically acceptable acid addition salt thereof.

6. A pharmaceutical composition comprising a pharmaceutically effective amount of a compound according to any of Claims 1-3 and a pharmaceutically acceptable carrier.

7. The use of a compound according to any of Claims 1-3 for the manufacture of a medicament for the treatment or prophylaxis of vascular disease.

**Claims for the following Contracting State : ES**

1. A method for preparing a pharmaceutical composition comprising the step of mixing a cis naphthothiazepine derivative of the formula:

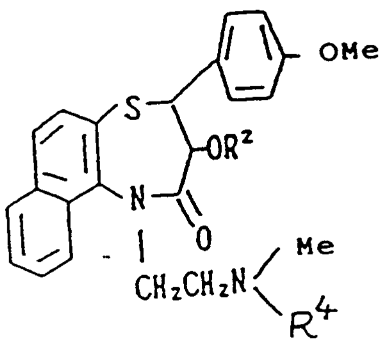

wherein $R^2$ is a hydrogen atom or an acetyl group, and $R^4$ is a methyl group, or a pharmaceutically acceptable acid addition salt thereof and a pharmaceutically acceptable carrier.

2. A method according to claim 1, wherein the cis naphthothiazepine derivative is (±)-Cis-2-(4-methoxyphenyl)-3-acetoxy-5-[2-(dimethylamino)ethyl]-2,3-dihydro-naphtho[2,1-b][1,5] thiazepin-4(5H)-one, or a pharmaceutically acceptable acid addition salt thereof.

3. A method according to claim 1, wherein the cis naphthothiazepine derivative is (±)-Cis-2-(4-methoxyphenyl)-3-hydroxy-5-[2-(dimethylamino)ethyl]-2,3-dihydro-naphtho[2,1-b][1,5] thiazepin-4(5H)-one, or a pharmaceutically acceptable acid addition salt thereof.

4. A process for preparing Cis-2-(4-methoxyphenyl)-3-hydroxy-5-[2-(dimethylamino)ethyl]-2,3-dihydronaphtho [2,1-b][1,5]thiazepin-4(5H)-one, or a pharmaceutically acceptable acid addition salt thereof, which comprises condensing Cis-2-(4-methoxyphenyl)-3-hydroxy-2,3-dihydro-naphtho[2,1-b][1,5]-thiazepin-4(5H)-one, or a salt thereof with 2-(dimethylamino)ethyl chloride, or a salt thereof, and if required, further converting the product into a pharmaceutically acceptable acid addition salt thereof.

5. A process for preparing Cis-2-(4-methoxyphenyl)-3-acetoxy-5-[2-(dimethylamino)ethyl]-2,3-dihydronaphtho [2,1-b][1,5]thiazepin-4(5H)-one, or a pharmaceutically acceptable acid addition salt thereof, which comprises condensing Cis-2-(4-methoxyphenyl)-3-hydroxy-5-[2-(dimethylamino)ethyl]-2,3-dihydro-naphtho[2,1-b](1,5] thiazepin-4(5H)-one, or a salt thereof with acetic acid, or a reactive derivative thereof, and if required, further converting the product into a pharmaceutically acceptable acid addition salt thereof.

6. The use of a cis naphthothiazepine derivative of the formula:

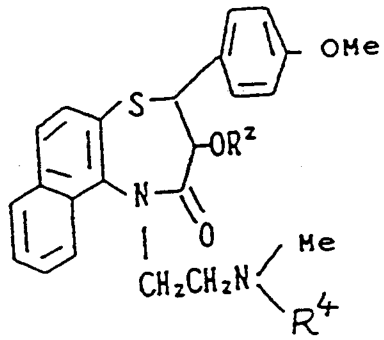

wherein $R^2$ is a hydrogen atom or an acetyl group, and $R^4$ is a methyl group, or a pharmaceutically acceptable acid addition salt thereof for the manufacture of a medicament for the treatment or prophylaxis of vascular disease.

7. The use according to Claim 6, wherein the cis naphthothiazepine derivative is (±)-Cis-2-(4-methoxyphenyl)-3-acetoxy-5-[2-(dimethylamino)ethyl]-2,3-dihydro-naphtho[2,1-b][1,5]thiazepin-4(5H)-one, or a pharmaceutically acceptable acid addition salt thereof.

8. The use according to Claim 6, wherein the cis naphthothiazepine derivative is (±)-Cis-2-(4-methoxyphenyl)-3-hydroxy-5-[2-(dimethylamino)ethyl]-2,3-dihydro-naphtho[2,1-b][1,5]thiazepin-4(5H)-one, or a pharmaceutically acceptable acid addition salt thereof.

**Claims for the following Contracting State : GR**

1. The use of a cis naphthothiazepine derivative of the formula:

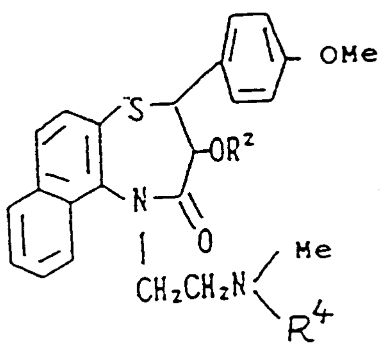

wherein $R^2$ is a hydrogen atom or an acetyl group, and $R^4$ is a methyl group, or a pharmaceutically acceptable acid addition salt thereof for the manufacture of a medicament for the treatment or prophylaxis of vascular disease.

2. The use according to Claim 1, wherein the cis naphthothiazepine derivative is (±)-Cis-2-(4-methoxyphenyl)-3-acetoxy-5-[2-(dimethylamino)ethyl]-2,3-dihydro-naphtho[2,1-b][1,5]thiazepin-4(5H)-one, or a pharmaceutically acceptable acid addition salt thereof.

3. The use according to Claim 1, wherein the cis naphthothiazepine derivative is (±)-Cis-2-(4-methoxyphenyl)-3-hydroxy-5-[2-(dimethylamino)ethyl]-2,3-dihydro-naphtho[2,1-b][1,5]thiazepin-4(5H)-one, or a pharmaceutically acceptable acid addition salt thereof.

4. A process for preparing Cis-2-(4-methoxyphenyl)-3-hydroxy-5-[2-(dimethylamino)ethyl]-2,3-dihydro-naptho[2,1-b][1,5]thiazepin-4(5H)-one or a pharmaceutically acceptable acid addition salt thereof, which

comprises condensing Cis-2-(4-methoxyphenyl)-3-hydroxy-2,3-dihydro-naptho[2,1-b][1,5]thiazepin-4(5)-one, or a salt thereof with 2-(dimethylamino)ethyl chloride, or a salt thereof, and if required, further converting the product into a pharmaceutically acceptable acid addition salt thereof.

**5.** A process for preparing Cis-2-(4-methoxyphenyl)-3-acetoxy5-[2-(dimethylamino)ethyl]-2,3-dihydro-naphtho[2,1-b][1,5]thiazepin-4(5H)-one, or a pharmaceutically acceptable acid addition salt thereof, which comprises condensing Cis-2-(4-methoxyphenyl)-3-hydroxy-5-[2(dimethylamino)ethyl]-2,3-dihydro-naphtho[2.1-b][1,5]thiazepin-4(5H)one, or a salt thereof with acetic acid, or a reactive derivative thereof, and if required, further converting the product into a pharmaceutically acceptable acid addition salt thereof.

**Patentansprüche**
**Patentansprüche für folgende Vertragsstaaten : AT, BE, CH, DE, FR, GB, IT, LI, LU, NL, SE**

**1.** Cis-Naphthothiazepin-Derivat der Formel

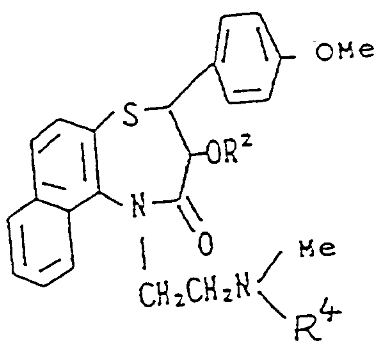

worin $R^2$ ein Wasserstoffatom oder eine Acetylgruppe ist und $R^4$ eine Methylgruppe ist, oder eines seiner pharmazeutisch zulässigen Säureadditionssalze.

**2.** Cis-Naphthothiazepin-Derivat gemäß Anspruch 1, welches (±)-cis-2-(4-Methoxyphenyl)-3-acetoxy-5-[2-(dimethylamino)-ethyl]-2,3-dihydro-naphtho[2,1-b][1,5]-thiazepin-4(5h)-on ist, oder eines seiner pharmazeutisch zulässigen Säureadditionssalze.

**3.** Cis-Naphthothiazepin-Derivat gemäß Anspruch 1, das (±)-cis-2-(4-Methoxyphenyl)-3-hydroxy-5-[2-(dimethylamino)-ethyl]-2,3-dihydro-naphtho[2,1-b][1,5]thiazepin-4(5H)-on ist, oder eines seiner pharmazeutisch zulässigen Säureadditionssalze.

**4.** Verfahren zur Herstellung eines cis-Naphthothiazepin-Derivats gemäß Anspruch 1, das cis-2-(4-Methoxyphenyl)-3-hydroxy-5[2-(dimethylamino)ethyl]-2,3-dihydro-naphthol[2,1-b][1,5]thiazepin-4(5H)-on oder eines seiner pharmazeutisch zulässigen Säureadditionssalze ist, umfassend die Kondensation von cis-2-(4-Methoxyphenyl)-3-hydroxy-2,3-dihydro-naphtho[2,1-b] [1,5]thiazepin-4(5H)-on oder eines Salzes davon mit 2-(Dimethylamino)ethylchlorid oder einem Salz davon und, falls gewünscht, weiterhin Umwandlung des Produkts in eines seiner pharmazeutisch zulässigen Säureadditionssalze.

**5.** Verfahren zur Herstellung eines cis-Naphthothiazepin-Derivats gemäß Anspruch 1, das cis-2-(4-Methoxyphenyl)-3-acetoxy-5-[2-(dimethylamino)-ethyl]-2,3-dihydro-naphtho[2,1-b][1,5]thiazepin-4(5H)-on oder ein pharmazeutisch zulässiges Säureadditionssalz davon ist, umfassend die Kondensation von cis-2-(4-Methoxyphenyl)-3-hydroxy-5-[2-(dimethylamino)-ethyl]-2,3-dihydro-naphtho-[2,1-b][1,5]thiazepin-4(5H)-on oder eines Salzes davon mit Essigsäure oder einem reaktiven Derivat davon, und falls gewünscht, weiterhin Umwandlung des Produkts in eines seiner pharmazeutisch zulässigen Säureadditionssalze.

**6.** Pharmazeutische Zusammensetzung, umfassend eine pharmazeutisch wirksame Menge einer Verbindung gemäß einem der Ansprüche 1 bis 3 und einen pharmazeutisch zulässigen Träger.

7. Verwendung einer Verbindung gemäß einem der Ansprüche 1 bis 3 zur Herstellung eines Medikaments zur Behandlung oder Prophylaxe von Gefäßkrankheiten.

**Patentansprüche für folgenden Vertragsstaat : ES**

1. Verfahren zur Herstellung einer pharmazeutischen Zusammensetzung, umfassend den Schritt der Mischung eines cis-Naphthothiazepin-Derivats der Formel:

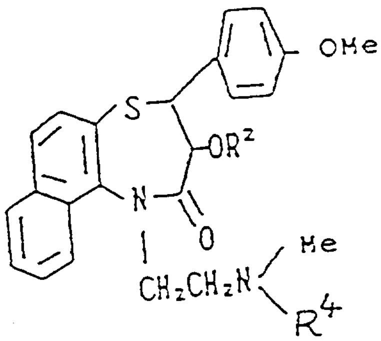

worin $R^2$ ein Wasserstoffatom oder eine Acetylgruppe ist, und $R^4$ eine Methylgruppe ist, oder eines seiner pharmazeutisch zulässigen Säureadditionssalze und eines pharmazeutisch zulässigen Trägers.

2. Verfahren gemäß Anspruch 1, dadurch **gekennzeichnet**, daß das cis-Naphthothiazepin-Derivat (±)-cis-2-(4-Methoxyphenyl)-3-acetoxy-5-[2-(dimethylamino)-ethyl]-2,3-dihydro-naphtho[2,1-b][1,5]thiazepin-4-(5H)-on oder ein pharmazeutisch zulässiges Säureadditionssalz davon ist.

3. Verfahren gemäß Anspruch 1, dadurch **gekennzeichnet**, daß das cis-Naphthothiazepin-Derivat (±)-cis-2-(4-Methoxyphenyl)-3-hydroxy-5-[2-(dimethylamino)-ethyl]-2,3-dihydro-naphtho[2,1-b][1,5]thiazepin-4-(5H)-on oder eines seiner pharmazeutisch zulässigen Säureadditionssalze ist.

4. Verfahren zur Herstellung von cis-2-(4-Methoxyphenyl)-3-hydroxy-5-[2-(dimethylamino)-ethyl]-2,3-dihydro-naphtho[2,1-b][1,5]thiazepin-4(5H)-on oder einem pharmazeutisch zulässigen Säureadditionssalz davon, umfassend die Kondensation von cis-2-(4-Methoxyphenyl)-3-hydroxy-2,3-dihydro-naphtho-[2,1-bl[1,5]thiazepin-4(5H)-on oder eines Salzes davon mit 2-(Dimethylamino)ethylchlorid oder einem Salz davon und, falls gewünscht, weiterhin Umwandeln des Produkts in eines seiner pharmazeutisch zulässigen Säureadditionssalze.

5. Verfahren zur Herstellung von cis-2-(4-Methoxyphenyl)-3-acetoxy-5-[2(dimethylamino)-ethyl]-2,3-dihydro -naphtho[2,1-b][1,5]thiazepin-4(5H)-on oder einem pharmazeutisch zulässigen Säureadditionssalz davon, umfassend die Kondensation von cis-2-(4-Methoxyphenyl)-3-hydroxy-5-[2-(dimethylamino)-ethyl]-2,3-dihydro-naphtho[2,1-b][2,5]thiazepin-4[5H)-on oder einem Salz davon mit Essigsäure oder einem reaktiven Derivat davon und, falls gewünscht, weiterhin Umwandeln des Produkts in eines seiner pharmazeutisch zulässigen Säureadditionssalze.

**6.** Verwendung eines cis-Naphthothiazepin-Derivats der Formel

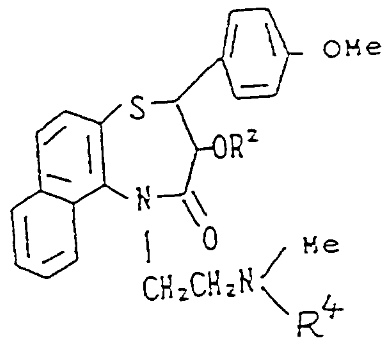

worin $R^2$ ein Wasserstoffatom oder eine Acetylgruppe ist und $R^4$ eine Methylgruppe ist, oder eines seiner pharmazeutisch zulässigen Säureadditionssalze zur Herstellung eines Medikaments zur Behandlung oder Prophylaxe von Gefäßkrankheiten.

**7.** Verwendung gemäß Anspruch 6, dadurch **gekennzeichnet**, daß das cis-Naphthothiazepin-Derivat (±)-cis-2-(4-Methoxyphenyl)-3-acetoxy-5-[2-(dimethylamino)ethyl]-2,3-dihydronaphtho-[2,1-b][1,5]thiazepin-4(5H)-on oder ein pharmazeutisch zulässiges Säureadditionssalz davon ist.

**8.** Verwendung gemäß Anspruch 6, dadurch **gekennzeichnet**, daß das cis-Naphthothiazepin-Derivat (±)-cis-2-(4-Methoxyphenyl)-3-hydroxy-5-[2-(dimethylamino)ethyl]-2,3-dihydronaphtho-[2,1-b][1,5]thiazepin-4(5H)-on oder ein pharmazeutisch zulässiges Säureadditionssalz davon ist.

**Patentansprüche für folgenden Vertragsstaat : GR**

**1.** Verwendung eines cis-Naphthothiazepin-Derivats der Formel

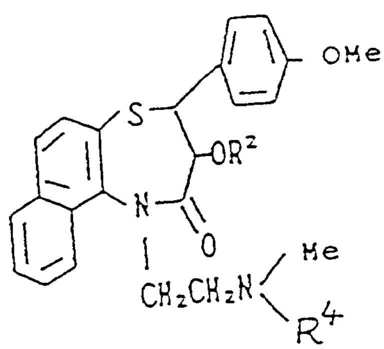

worin $R^2$ ein Wasserstoffatom oder eine Acetylgruppe ist und $R^4$ eine Methylgruppe ist, oder eines pharmazeutisch zulässigen Säureadditionssalzes davon zur Herstellung eines Medikaments zur Behandlung oder Prophylaxe von Gefäßkrankheiten.

**2.** Verwendung gemäß Anspruch 1, dadurch **gekennzeichnet**, daß das cis-Naphthothiazepin-Derivat (±)-cis-2-(4-Methoxyphenyl)-3-acetoxy-5-[2-(dimethylamino)ethyl]-2,3-dihydronaphtho-[2,1-b][1,5]thiazepin-4(5H)-on oder ein pharmazeutisch zulässiges Säureadditionssalz davon ist.

**3.** Verwendung gemäß Anspruch 1, dadurch **gekennzeichnet,** daß das cis-Naphthothiazepin-Derivat (±)-cis-2-(4-Methoxyphenyl)-3-hydroxy-5-[2-(dimethylamino)ethyl]-2,3-dihydronaphtho-[2,1-b][1,5]thiazepin-4(5H)-on oder eines seiner pharmazeutisch zulässigen Säureadditionssalze ist.

**4.** Verfahren zur Herstellung von cis-2-(4-Methoxyphenyl)-3-hydroxy-5-[2-(dimethylamino)-ethyl]-2,3-dihydro-naphtho[2,1-b][1,5]thiazepin-4(5H)-on oder eines pharmazeutisch zulässigen Säureadditionssalzes davon, umfassend die Kondensation von cis-2-(4-Methoxyphenyl)-3-hydroxy-2,3-dihydro-naphtho [2,1-b][1,5]thiazepin-4(5H)-on oder eines Salzes davon mit 2-(Dimethylamino)ethylchlorid oder einem Salz davon und, falls gewünscht, weiterhin Umwandeln des Produkts in eines seiner pharmazeutisch zulässigen Säureadditionssalze.

**5.** Verfahren zur Herstellung von cis-2-(4-Methoxyphenyl)-3-acetoxy-5-[2-(dimethylamino)-ethyl]-2,3-dihydro-naphtho[2,1-b][1,5]thiazepin-4(5H)-on oder eines pharmazeutisch zulässigen Säureadditionssalzes davon, umfassend die Kondensation von cis-2-(4-Methoxyphenyl)-3-hydroxy-5-[2-(dimethylamino)-ethyl]-2,3-dihydro-naphtho[2,1-b][1,5]thiazepin-4(5H)-on oder eines Salzes davon mit Essigsäure oder einem reaktiven Derivat davon und, falls gewünscht, weiterhin Umwandeln des Produkts in eines seiner pharmazeutisch zulässigen Säureadditionssalze.

**Revendications**

**Revendications pour les Etats contractants suivants : AT, BE, CH, DE, FR, GB, IT, LI, LU, NL, SE**

**1.** Dérivé de cis-naphtothiazépine de formule :

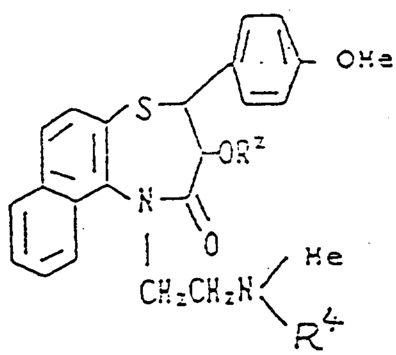

dans laquelle $R^2$ représente un atome d'hydrogène ou un groupe acétyle et $R^4$ représente un groupe méthyle, et sels d'addition d'acide pharmaceutiquement acceptables d'un tel dérivé.

**2.** Dérivé de cis-naphtothiazépine conforme à la revendication 1, qui est la (±)-cis-2-(4-méthoxyphényl)-3-acétoxy-5-[2-(diméthylamino)éthyl]-2,3-dihydro-naphto-[2,1-b][1,5]-thiazépine-4(5H)-one, ou l'un de ses sels d'addition d'acide pharmaceutiquement acceptables.

**3.** Dérivé de cis-naphtothiazépine conforme à la revendication 1, qui est la (±)-cis-2-(4-méthoxyphényl)-3-hydroxy-5-[2-(diméthylamino)éthyl]-2,3-dihydro-naphto-[2,1-b][1,5]-thiazépine-4(5H)-one, ou l'un de ses sels d'addition d'acide pharmaceutiquement acceptables.

**4.** Procédé pour préparer un dérivé de cis-naphtothiazépine conforme à la revendication 1, qui est la cis-2-(4-méthoxyphényl)-3-hydroxy-5-[2-(diméthylamino)-éthyl]-2,3-dihydro-naphto-[2,1-b][1,5]-thiazépine-4-(5H)-one, ou l'un de ses sels d'addition d'acide pharmaceutiquement acceptables, qui comporte la condensation de la cis-2-(4-méthoxyphényl)-3-hydroxy-2,3-dihydro-naphto[2,1-b][1,5]-thiazépine-4(5H)-one, ou de l'un de ses sels, avec du chlorure de 2-(diméthylamino)-éthyle ou avec l'un de ses sels, et si nécessaire, la conversion ultérieure du produit obtenu en l'un de ses sels d'addition d'acide pharmaceutiquement acceptables.

**5.** Procédé pour préparer un dérivé de cis-naphtothiazépine conforme à la revendication 1, qui est la cis-2-(4-méthoxyphényl)-3-acétoxy-5-[2-(diméthylamino)-éthyl]-2,3-dihydro-naphto-[2,1-b][1,5]-thiazépine-4-(5H)-one, ou l'un de ses sels d'addition d'acide pharmaceutiquement acceptables, qui comporte la condensation de la cis-2-(4-méthoxyphényl)-3-hydroxy-5-[2-(diméthylamino)-éthyl]-2,3-dihydro-naphto-[2,1-b][1,5]-thiazépine-4(5H)-one, ou de l'un de ses sels, avec de l'acide acétique ou avec l'un de ses dérivés réactifs, et si nécessaire, la conversion ultérieure du produit obtenu en l'un de ses sels d'addition d'acide pharmaceutiquement acceptables.

6. Composition pharmaceutique comprenant une quantité pharmaceutiquement efficace d'un composé conforme à l'une quelconque des revendications 1 à 3 et un véhicule pharmaceutiquement acceptable.

7. Utilisation d'un composé conforme à l'une quelconque des revendications 1 à 3 pour la fabrication d'un médicament destiné au traitement ou à la prophylaxie de maladies vasculaires.

**Revendications pour l'Etat contractant suivant : ES**

1. Procédé de préparation d'une composition pharmaceutique comprenant l'étape consistant à mélanger un dérivé de cis-naphtothiazépine de formule :

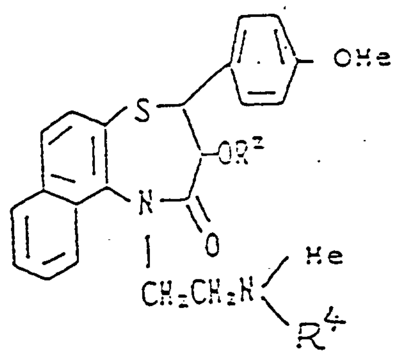

dans laquelle $R^2$ représente un atome d'hydrogène ou un groupe acétyle et $R^4$ représente un groupe méthyle, ou un sel d'addition d'acide pharmaceutiquement acceptable d'un tel dérivé, et un véhicule pharmaceutiquement acceptable.

2. Procédé selon la revendication 1, dans lequel le dérivé de cis-naphtothiazépine est la (±)-cis-2-(4-méthoxyphényl)-3-acétoxy-5-[2-(diméthylamino)éthyl]-2,3-dihydro-naphto-[2,1-b][1,5]-thiazépine-4(5H)-one, ou l'un de ses sels d'addition d'acide pharmaceutiquement acceptables.

3. Procédé selon la revendication 1, dans lequel le dérivé de cis-naphtothiazépine est la (±)-cis-2-(4-méthoxyphényl)-3-hydroxy-5-[2-(diméthylamino)éthyl]-2,3-dihydro-naptho-[2,1-b][1,5]-thiazépine-4(5H)-one, ou l'un de ses sels d'addition d'acide pharmaceutiquement acceptables.

4. Procédé pour préparer la cis-2-(4-méthoxyphényl)-3-hydroxy-5-[2-(diméthylamino)-éthyl]-2,3-dihydro-naptho-[2,1-b][1,5]-thiazépine-4(5H)-one, ou l'une de ses sels d'addition d'acide pharmaceutiquement acceptables, qui comporte la condensation de la cis-2-(4-méthoxyphényl)-3-hydroxy-2,3-dihydro-naphto[2,1-b][1,5]-thiazépine-4(5H)-one, ou de l'un de ses sels, avec du chlorure de 2-(diméthylamino)-éthyle ou avec l'un de ses sels, et si nécessaire, la conversion ultérieure du produit obtenu en l'un de ses sels d'addition d'acide pharmaceutiquement acceptables.

5. Procédé pour PréParer la cis-2-(4-méthoxyphényl)-3-acétoxy-5-[2-(diméthylamino)-éthyl]-2,3-dihydro-naptho-[2,1-b][1,5]-thiazépine-4(5H)-one, ou l'une de ses sels d'addition d'acide pharmaceutiquement acceptables, qui comporte la condensation de la cis-2-(4-méthoxyphényl)-3-hydroxy-5-[2-(diméthylamino)-éthyl]-2,3-dihydro-naptho-[2,1-b][1,5]-thiazépine-4(5H)-one, ou l'une de ses sels, avec de l'acide acétique ou avec l'un de ses dérivés réactifs, et si nécessaire, la conversion ultérieure du produit obtenu en l'un de ses sels d'addition d'acide pharmaceutiquement acceptables.

**6.** Utilisation d'un dérivé de cis-naphtothiazépine de formule :

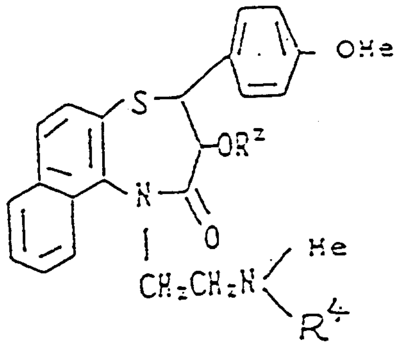

dans laquelle $R^2$ représente un atome d'hydrogène ou un groupe acétyle et $R^4$ représente un groupe méthyle, ou d'un sel d'addition d'acide pharmaceutiquement acceptable d'un tel dérivé, pour la fabrication d'un médicament destiné au traitement ou à la prophylaxie de maladies vasculaires.

**7.** Utilisation conforme à la revendication 6, dans laquelle le dérivé de cis-naphtothiazépine est la (±)-cis-2-(4-méthoxyphényl)-3-acétoxy-5-[2-(diméthylamino)éthyl]-2,3-dihydro-naptho-[2,1-b][1,5]-thiazépine-4-(5H)-one, ou l'un de ses sels d'addition d'acide pharmaceutiquement acceptables.

**8.** Utilisation conforme à la revendication 6, dans laquelle le dérivé de cis-naphtothiazépine est la (±)-cis-2-(4-méthoxyphényl)-3-hydroxy-5-[2-(diméthylamino)-éthyl]-2,3-dihydro-naptho-[2,1-b][1,5]-thiazépine-4-(5H)-one, ou l'un de ses sels d'addition d'acide pharmaceutiquement acceptables.

**Revendications pour l'Etat contractant suivant : GR**

**1.** Utilisation d'un dérivé de cis-naphtothiazépine de formule :

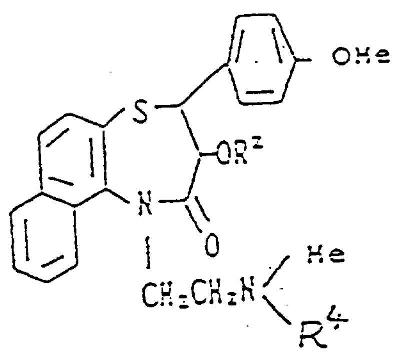

dans laquelle $R^2$ représente un atome d'hydrogène ou un groupe acétyle et $R^4$ représente un groupe méthyle, ou d'un sel d'addition d'acide pharmaceutiquement acceptable d'un tel dérivé, pour la fabrication d'un médicament destiné au traitement ou à la prophylaxie de maladies vasculaires.

**2.** Utilisation conforme à la revendication 1, dans laquelle le dérivé de cis-naphtothiazépine est la (±)-cis-2-(4-méthoxyphényl)-3-acétoxy-5-[2-(diméthylamino)éthyl]-2,3-dihydro-naptho-[2,1-b][1,5]-thiazépine-4-(5H)-one, ou l'un de ses sels d'addition d'acide pharmaceutiquement acceptables.

**3.** Utilisation conforme à la revendication 1, dans laquelle le dérivé de cis-naphtothiazépine est la (±)-cis-2-(4-méthoxyphényl)-3-hydroxy-5-[2-(diméthylamino)éthyl]-2,3-dihydro-naptho-[2,1-b][1,5]-thiazépine-4-(5H)-one, ou l'un de ses sels d'addition d'acide pharmaceutiquement acceptables.

**4.** Procédé pour préparer la cis-2-(4-méthoxy-phényl)-3-hydroxy-5-[2-(diméthylamino)-éthyl]-2,3-dihydro-naphto-[2,1-b][1,5]-thiazépine-4(5H)-one, ou l'un de ses sels d'addition d'acide pharmaceutiquement

acceptables, qui comporte la condensation de la cis-2-(4-méthoxyphényl)-3-hydroxy-2,3-dihydro-naphto[2,1-b][1,5]-thiazépine-4(5H)-one, ou de l'un de ses sels, avec du chlorure de 2-(diméthylamino)-éthyle ou avec l'un de ses sels, et si nécessaire, la conversion ultérieure du produit obtenu en l'un de ses sels d'addition d'acide pharmaceutiquement acceptables.

**5.** Procédé pour préparer la cis-2-(4-méthoxyphényl)-3-acétoxy-5-[2-(diméthylamino)-éthyl)-2,3-dihydro-naptho-[2,1-b][1,5]-thiazépine-4(5H)-one, ou l'une de ses sels d'addition d'acide pharmaceutiquement acceptables, qui comporte la condensation de la cis-2-(4-méthoxyphényl)-3-hydroxy-5-[2-(diméthylamino)-éthyl]-2,3-dihydro-naptho-[2,1-b][1,5]-thiazépine-4(5H)-one, ou de l'un de ses sels, avec de l'acide acétique ou avec l'un de ses dérivés réactifs, et si nécessaire, la conversion ultérieure du produit obtenu en l'un de ses sels d'addition d'acide pharmaceutiquement acceptables.